(19) Europäisches Patentamt
European Patent Office
Office européen des brevets

(11) Publication number: **0 093 550**
**B1**

(12) # EUROPEAN PATENT SPECIFICATION

(45) Date of publication of patent specification: **29.01.86**

(51) Int. Cl.$^4$: **C 07 C 69/96,** C 07 C 68/08, C 08 F 2/00, C 08 F 118/12

(21) Application number: **83302289.0**

(22) Date of filing: **22.04.83**

(54) **Inhibited monomeric compositions of polyol(allyl carbonate), method for their preparation and method for their polymerization.**

(30) Priority: 26.04.82 US 371404

(43) Date of publication of application:
**09.11.83 Bulletin 83/45**

(45) Publication of the grant of the patent:
**29.01.86 Bulletin 86/05**

(84) Designated Contracting States:
**BE DE FR GB IT NL**

(56) References cited:
**EP-A-0 035 304**
**DE-B-1 946 823**

(73) Proprietor: **PPG INDUSTRIES, INC.**
**One PPG Place**
**Pittsburgh Pennsylvania 15272 (US)**

(72) Inventor: **Leatherman, Ivan Roger**
**298 Crestwood Avenue**
**Wadsworth Ohio 44281 (US)**

(74) Representative: **Shipton, Gordon Owen et al**
**W.P. THOMPSON & CO. Coopers Building**
**Church Street**
**Liverpool L1 3AB (GB)**

EP 0 093 550 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to inhibited monomeric compositions of polyol(allyl carbonate), method for their preparation and method for their polymerization.

Polyol(allyl carbonate) monomers polymerize through sites of allylic unsaturation. The thermodynamics and kinetics of allyl polymerization indicate a high activation energy is required for the polymerization, and that a high degree of chain transfer and chain termination occurs. These have the practical effect of making allyl monomers, e.g., allyl carbonate monomers, relatively stable to high temperatures during shipping and storage.

However, it has been found that compositions containing a major portion of uncatalyzed polyol(allyl carbonate) monomer, especially compositions thickened by having dispersed polymer therein, are subject to thickening. This is especially so at temperatures above about 45 degrees Centigrade. By compositions containing a major portion of uncatalyzed polyol(allyl carbonate) monomer we mean compositions having the properties of the monomer.

It has now been found that this tendency toward thermally induced thickening may be reduced or even eliminated by the addition of a dihydroxy aromatic compound, dioxo aromatic compound, or derivative thereof e.g. alkyl ethers or benzyl ethers, to the composition of polyol(allyl carbonate) monomer.

In the accompanying drawings:

Figure I is a graphical representation of the data in Examples I and II, showing the viscosity versus time behaviour of uninhibited diethylene glycol bis(allyl carbonate) and diethylene glycol bis(allyl carbonate) inhibited with various levels of hydroquinone monomethyl ether.

Figure II is a graphical representation of the data in Examples I and III, showing the viscosity versus time behaviour of uninhibited diethylene glycol bis(allyl carbonate) and diethylene glycol bis(allyl carbonate) inhibited with various levels of tertiary butyl catechol.

Figure III is a graphical representation of the data in Examples I and IV showing the viscosity versus time behaviour of uninhibited diethylene glycol bis(allyl carbonate) and diethylene glycol bis(allyl carbonate) inhibited with various levels of hydroquinone.

Figure IV is a graphical representation of the data in Examples I and V showing the viscosity versus time behaviour of uninhibited diethylene glycol bis(allyl carbonate) and diethylene glycol bis(allyl carbonate) inhibited with various levels of quinone.

It has now been found that the presence of low levels, e.g., less than 20 parts per million of polymerization inhibitors e.g., dioxoaromatics and dihydroxyaromatics, substantially reduce, and even eliminate the tendency of compositions of polyol(allyl carbonate) monomer to thicken.

While the mechanism of the thickening of polyol(allyl carbonate) compositions is not fully understood, and while not wishing to be bound by this explanation, it is believed that thermally initiated polymerization may occur at sites of allyl unsaturation. It is possible that the presence of trace amounts of glycols, carbonates, allyl carbonates, glycol carbonates, and the like may enhance the postulated non-catalytic, thermal polymerization.

According to the present invention there is provided a composition comprising monomeric polyol(allyl carbonate) and a viscosity stabilizing amount of polymerization inhibitor chosen from the group consisting of dihydroxybenzenes, dioxo benzenes, hydroquinone monomethyl ether, hydroquinone monoethyl ether, hydroquinone benzyl ether, and mixtures thereof in the composition.

The invention also includes a method of inhibiting the thermally induced thickening of compositions containing monomeric poly(allyl carbonate) comprising providing a viscosity stabilizing amount of a polymerization inhibitor chosen from the group consisting of dihydroxybenzenes, dioxo benzenes, hydroquinone monomethyl ether, hydroquinone monoethyl ether, hydroquinone benzyl ether, and mixtures thereof in the composition.

The invention further includes a method of polymerizing a composition of polyol(allyl carbonate) monomer comprising providing a viscosity stabilizing amount of a polymerization inhibitor chosen from the group consisting of dihydroxybenzenes, dioxo benzenes, hydroquinone monomethyl ether, hydroquinone monoethyl ether, hydroquinone benzyl ether, and mixtures thereof in the composition whereby to inhibit the thermally induced thickening thereof, and thereafter, without removing the polymerization inhibitor, adding a free radical polymerization initiator to the composition and heating the composition whereby to polymerize the composition.

As used herein, the term "polymerization inhibitor" refers to an additive to the monomeric polyol(allyl carbonate) that reduces and even prevents uncatalyzed, thermal polymerization of polyol(allyl carbonate) to form poly[polyol bis(allyl carbonate)] under conditions of storage that otherwise would permit thermal polymerization. The preferred, inhibitors are those which when added to the monomeric polyol(allyl carbonate) at concentrations of less than 20 parts per million, basis monomer, maintain the viscosity of monomeric polyol bis(allyl carbonate) stored at a temperature of about 45 degrees Centigrade below twice the original viscosity after four months, below four times the original viscosity after six months, below eight times the original viscosity after eight months, and below sixteen times the original viscosity after ten months. More preferred inhibitors are those that, when present at the 5 parts per million level, maintain the viscosity within three times the original viscosity after ten months of storage at 45 degrees Centigrade. Especially preferred are those inhibitors that, when present at the 5 parts per million level, maintain the

viscosity within twenty percent of the original value after ten months of storage at 45 degrees Centigrade.

As used herein, the term "uncatalyzed polymerization" includes the thickening of the polyol(allyl carbonate) upon standing without the addition of free radical polymerization initiator thereto. As used herein, the term "catalyzed polymerization" means the polymerization of the polyol(allyl carbonate) after the addition of free radial polymerization initiator thereto.

The inhibitors of the present invention are characterized by substantially complete effectiveness in preventing thickening of diol bis(allyl carbonate) compositions at temperatures of at least 45 degrees Centigrade for time periods of at least ten months, at additive concentrations of 20 parts per million or less of inhibitor, basis diol bis(allyl carbonate), and generally from about 2 to about 20 parts per million. By "substantially complete effectiveness" is meant that the viscosity of the additive containing diol bis(allyl carbonate) monomer is within ten percent of the original viscosity after ten months exposure to temperatures of 45 degrees Centigrade.

The inhibitors of the invention are further characterized by the ability to permit polymerization to normal physical properties, e.g. abrasion resistance, impact strength, Barcol hardness, optical clarity, yellowness, and dyeability, with normal cure cycles, polymerization initiators, and polymerization initiator concentrations.

Exemplary of the inhibitors of the invention are the monoaromatics, e.g. the quinones, the hydroquinones, and catechols.

Examples of the preferred inhibitors are dihydroxy benzenes, derivatives thereof, dioxo benzenes, derivatives thereof, and mixtures thereof the oxygens occupying positions that are either *ortho* or *para* to each other.

Exemplary dihydroxy benzenes and derivatives thereof are hydroquinone,

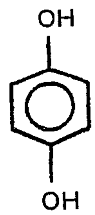

,

mono-tertiary butyl hydroquinone,

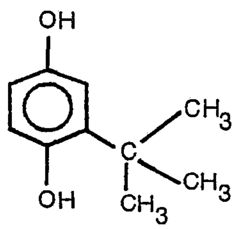

toluhydroquinone,

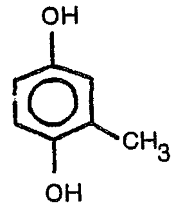

,

catechol,

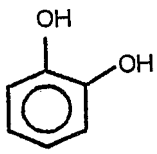

,

tertiary butyl catechol,

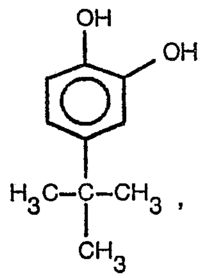

and the hydroquinone ethers,

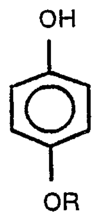

where R may be for example a methyl group, as in methyl hydroquinone monomethyl ether

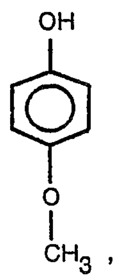

or R may be for example an ethyl group, as in hydroquinone monoethyl ether,

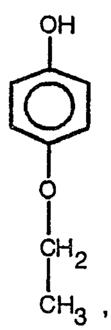

or an aromatic group as in hydroquinone benzyl ether,

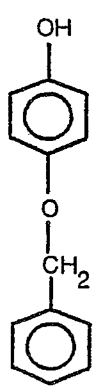

Exemplary dioxo benzenes are quinone, i.e., para-quinone,

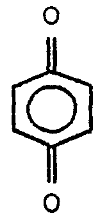,

ortho-quinone,

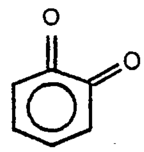,

and toluquinone

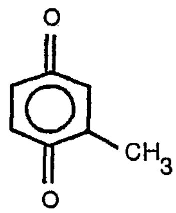.

Particularly preferred are the dihydroxy benzenes and derivatives thereof, e.g., hydroquinone, catechol, mono-tertiary butyl hydroquinone, toluhydroquinone, tertiary butyl catechol, hydroquinone monomethyl ether, hydroquinone monoethyl ether, and hydroquinone benzyl ether. Especially preferred are hydroquinone and hydroquinone monomethyl ether.

The disclosed method of combating the tendency of polyol(allyl carbonate) monomer compositions to gel or thicken on storage, e.g., at elevated temperatures, may be utilized with various polyol(allyl carbonate) monomer compositions, for example, diol bis(allyl carbonates), triol tris(allyl carbonates), tetra kis(allyl carbonates), and higher polyol(allyl carbonates).

Diol bis(allyl carbonate) monomers which may be inhibited by the method of this invention are normally linear, liquid allyl carbonates, and, most commonly, are aliphatic diol bis(allyl carbonates) e.g., glycol bis(allyl carbonate) compounds, in which the allyl groups may be substituted at the 2 position with a halogen, notably chlorine or bromine, or a 1 to 4 carbon alkyl group, generally a methyl or ethyl group, and the glycol group may be an alkylene, alkylene ether, alkylene polyether, alkylene carbonate, or alkylene polyether group having from 2 to 10 carbons and oxygens. These diol bis(allyl carbonate) monomers are represented by the formula:

$$R_1 - O - \overset{\overset{\displaystyle O}{\|}}{C} - O - R_2 - O - \overset{\overset{\displaystyle O}{\|}}{C} - O - R_3$$

where $R_1$ and $R_3$ are allyl or substituted allyl groups, and $R_2$ is derived from a diol $HOR_2OH$ and $R_2$ being a diol fragment selected from alkylene groups, alkylene ether groups, alkylene polyether groups, and alkylene polycarbonate groups as defined below. $R_1$ and $R_3$ are independently represented by the formula:

$$H_2C{=}\overset{\overset{\displaystyle R^o}{|}}{C}{-}CH_2{-}$$

where $R^o$ may be hydrogen, halogen, or a 1 to 4 carbon alkyl group. Specific examples of $R_1$ and $R_3$ are allyl, 2-chloroallyl, 2-bromallyl, 2-iodoallyl, 2-fluoroallyl, 2-methallyl, 2-ethylallyl, 2-isopropylallyl 2-n-propylallyl, and 2-n-butylallyl groups. Most commonly, $R_1$ and $R_3$ are allyl groups, $H_2C{=}CH{-}CH_2{-}$. Such compounds and methods for making them are disclosed in U.S. Patents 2,370,567 and 2,403,113.

Specific examples of $R_2$ are alkylene groups such as ethylene, trimethylene, methylethylene, tetramethylene, ethylethylene, pentamethylene, hexamethylene, 2-methylhexamethylene, octamethylene, and decamethylene groups, alkylene ether groups such as

$—CH_2—O—CH_2—$, $—CH_2CH_2—O—CH_2CH_2—$, $—CH_2—O—CH_2—CH_2—$, and

$—CH_2CH_2CH_2—O—CH_2CH_2CH_2—$,

alkylene polyether groups such as

$—CH_2CH_2—O—CH_2CH_2—O—CH_2CH_2—O—CH_2CH_2O—CH_2CH_2—$, and

$—CH_2—O—CH_2—$

groups, and alkylene carbonate groups such as

$CH_2CH_2—O—CO—O—CH_2CH_2$ and

$—CH_2CH_2—O—CH_2CH_2—O—CO—O—CH_2CH_2—OCH_2CH_2—$

groups. Most commonly, $R_2$ is

$—CH_2CH_2—$ or

$CH_2CH_2—O—CH_2CH_2—$.

Specific examples of diol bis(allyl carbonate) monomers in which the method herein contemplated is useful are ethylene glycol bis(2-chloroallyl carbonate), diethylene glycol bis(2-methyallyl carbonate), triethylene glycol bis(allyl carbonate), propylene glycol bis(2-ethylallyl carbonate), 1,3-propanediol bis(allyl carbonate), 1,3-butanediol bis(allyl carbonate), 1,4-butanediol bis(2-bromallyl carbonate), dipropylene glycol bis(allyl carbonate), trimethylene glycol bis(2-ethylallyl carbonate), and pentamethylene glycol bis(allyl carbonate).

Commercially important diol bis(allyl carbonate) monomers which may be inhibited or stabilized by the method herein contemplated are:

$$CH_2 = CH - CH_2 - O - \overset{\overset{O}{\|}}{C} - O - CH_2 - CH_2 - O - CH_2 - CH_2 - \overset{\overset{O}{\|}}{O} - C - O - CH_2CH = CH_2,$$

and $$CH_2 = CH - CH_2 - O - \overset{\overset{O}{\|}}{C} - O - CH_2 - CH_2 - O - \overset{\overset{O}{\|}}{C} - O - CH_2 - CH = CH_2$$

Alternatively, the method of this invention is useful in inhibiting thermal polymerization in aromatic bis(allyl carbonates), e.g., bisphenol-A bis(allyl carbonate) and ethoxylated bis phenol-A-bis(allyl carbonate).

Triol tris(allyl carbonates) which may be inhibited by the method of this invention are represented by the formula

$$R_4\ (O\overset{\overset{O}{\|}}{C}O—CH_2CH{=}CH_2)_n$$

where $R_4$ is an organic moiety chosen from the group consisting of moieties derived from polyols and extended polyols, most frequently a triol or extended triol where the hydroxyl groups of the precursor polyol $R_4(OH)_n$ are non-vicinal.

While the functionality is shown as tris functionality, it is to be understood that in higher polyols n is greater than 2, e.g., above about 2.2, representing a mixture of diols and higher polyols, to about 8 representing a derivative of trimeric pentaerythritol. By "non-vicinal" it is meant that the hydroxyl groups are not on adjacent carbons. Specific triol precursors useful in preparing the tris(allyl carbonate) materials useful in this invention are triols with primary or secondary hydroxyl groups. Triols having primary hydroxyl groups are preferred precursors. One such class of triols are 1,1,1-trimethylol alkanes. Also useful are extended trimethylol alkane tris(allyl carbonate) monomers such as lactone extended trimethylol alkanes and alkylene oxide extended trimethylol alkanes. By and "extended triol" is meant the reaction product having terminal hydroxyl groups of the triol and a suitable reactant, i.e., one that reacts with a triol to form another triol e.g., an alkylene oxide or a lactone. Typical lactone extended trimethylol alkanes

include ε-caprolactone extended trimethylol methane, ε-caprolactone extended trimethylol ethane, ε-caprolactone extended trimethylol propane, and ε-caprolactone extended trimethylol butane. Typical alkylene oxide extended triols include ethylene oxide extended trimethylol methane, ethylene oxide extended trimethylol ethane, ethylene oxide extended trimethylol propane, ethylene oxide extended trimethylol butane, propylene oxide extended trimethylol methane, propylene oxide extended trimethylol methane, propylene oxide extended trimethylol ethane and propylene oxide extended trimethylol butane.

The preferred polyols meeting these requirements have the general formula $R_4(OH)_n$ where n is greater than 2 up to about 8 and generally is about 3. $R_4$ can be, for example,

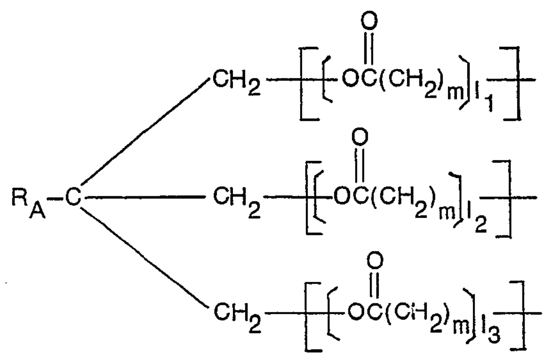

where $R_A$ is

$$H, -CH_3, -CH_2CH_3, -CH_2CH_2CH_3 \text{ or}$$

$$-CH_2CH_2CH_3,$$

and $l_1$, $l_2$ and $l_3$ are each integers from 0 to 5 and the sum of $l_1 + l_2 + l_3$ is 2 or more and generally from 2 to 8, although values as high as 15 are possible. The value of m depends on the lactone utilized to extend the polyol and is generally 4 or 5.

The chain extending lactone may be for example a delta lactone having the formula

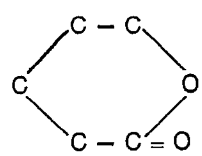

which can be substituted with hydrogen, methyl groups, or ethyl groups.

According to a still further exemplification, the chain extending lactone group can be an epsilon lactone having the formula:

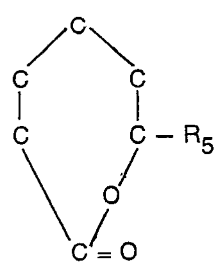

where $R_5$ is hydrogen, a methyl group, or an ethyl group and where $R_5$ can be on any of the carbons other than the carbonyl carbon. One exemplary triol is Union Carbide Corporation NIAX® PCP—0301 brand epsilon-caprolactone extended trimethylol propane.

According to a still further exemplification, $R_4$ can be

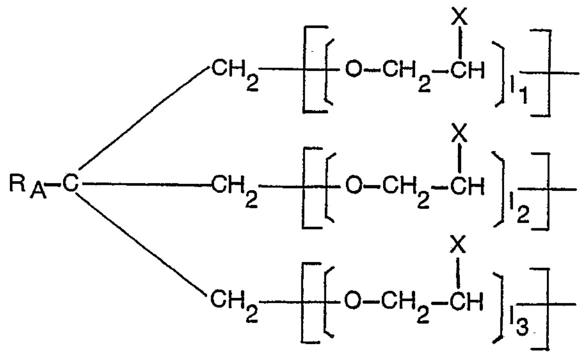

where $R_A$ is as defined previously, $l_1$, $l_2$ and $l_3$ are integers from 0 to 5 and the sum of $l_1 + l_2 + l_3$ is 2 or more and generally from 2 to 8, although values as high as about 15 are possible, and X is H or $CH_3$. The chain extenders may be ethylene oxide groups as exemplified by Upjon ISONOL® 93 ethylene oxide extended trimethylol propane. Alternatively, the extenders may be propylene oxide groups as in BASF = Wyandotte PLURACOL TP brand propoxylated trimethylol propane.

According to a still further exemplification, $R_4(OH)_3$ may be an extended glycerol, for example, ethylene oxide extended glycerol having the general formula:

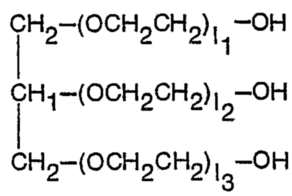

or propylene oxide extended glycerol having the formula:

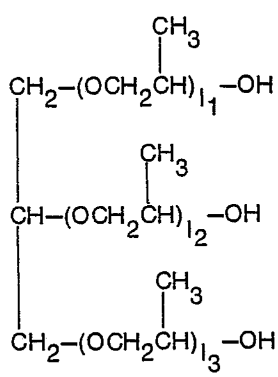

or a lactone extended glycerol having the formula:

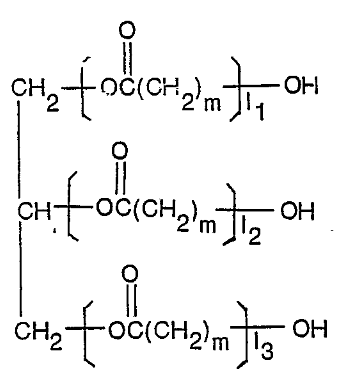

where m and $l_1$, $l_2$ and $l_3$ are as defined above. Typical propoxylated glycerines are DOW VORANOL 2025 brand propoxylated glycerine having a molecular weight of about 260 grams per gram mole, DOW

VORANOL 2070 brand propoxylated glycerine having a molecular weight of about 700 grams per gram mole, and BASF-Wyandotte PLURACOL GP730 brand propoxylated glycerine having a molecular weight of about 730 grams per mole.

While the compositions are referred to as being monomeric compositions of polyol(allyl carbonate), it is to be understood that they may be compositions of polyol(allyl carbonate) monomer having polymers dispersed therein, e.g., as described in the U.S. Patent No. 4346197 or with polymer and colorants dispersed therein, as described in European Patent Application No. 82306167.6, or with polymer and multifunctional acrylate monomers dispersed therein, as described in copending U.S. Patent No. 4,408,016.

The inhibitors herein contemplated are effective to substantially prevent the uncatalyzed thickening of the polyol(allyl carbonates) at low concentrations of additive. Moreover, the concentration of additive is low enough to avoid inhibition of the free radical catalyzed polymerization of polyol(allyl carbonate).

The polymerization of the polyol(allyl carbonate) composition is initiated by the creation of active centers, e.g., free radicals. Useful polymerization initiators are organic peroxy initiators. Examples of organic peroxy polymerization initiators are isobutyryl peroxide, di(2-ethylhexyl) peroxydicarbonate; acetyl cyclohexane sulfonyl peroxide; di(sec-butyl) peroxydicarbonate, diisopropyl peroxydicarbonate; 2,4-dichlorobenzoyl peroxide, t-butyl peroxypivalate, decanoyl peroxide, lauroyl peroxide, propionyl peroxide; 2,5-dimethyl-2,5-bis(2-ethyl hexylperoxy) hexane; acetyl peroxide; succinic acid peroxide; t-butyl peroxyoctoate; benzoyl peroxide; p-chlorobenzoyl peroxide; t-butyl peroxyisobutyrate; t-butyl peroxymaleic acid; bis(1-hydroxycyclohexyl) peroxide, 1-hydroxy-1'-hydroperoxy dicyclohexyl peroxide; t-butyl peroxyisopropyl carbonate; 2,5-dimethyl-2,5-bis(benzoylperoxy) hexane; t-butyl peroxyacetate; methyl ethyl ketone peroxides; di-t-butyl diperoxyphthalate and t-butyl peroxybenzoate.

According to one exemplification of the invention, 20 part per million of hydroquinone mono-methyl ether, basis monomeric composition, is added to industrial diethylene glycol bis-(allyl carbonate) monomer containing from 80 to 90 weight percent diethylene glycol bis(allyl carbonate) monomer, balance impurities in the monomer, e.g., diethylene glycol, diethylene glycol mono allyl carbonate, oligomeric compounds, diallyl carbonate, diethylene glycol polycarbonate, or diethylene glycol carbonate. The initial viscosity of the composition is from 15 to 20 centistokes. Aften ten months of storage at 45 degrees Centigrade the viscosity of the composition is still 15 to 20 centistokes.

Thereafter the composition is polymerized by the addition of 3.5 weight percent isopropyl peroxydicarbonate to the composition, and heating from 27 degrees Centigrade to 100 degrees Centigrade over a period of sixteen to twenty four hours. The resulting polymerizate is a rigid solid.

The following Examples are illustrative.

Example I

A test was conducted to determine the effect of heat on a monomeric composition of diethylene glycol bis(allyl carbonate).

A four ounce narrow mouth, screw top bottle was filled with PPG Industries, Inc. CR—39® diethylene glycol bis(allyl carbonate). The screw top was loosely fitted to allow for pressure relief, and the sample was placed in an air circulating oven at 45 degrees Centigrade. The sample was removed periodically for viscosity measurement. The viscosities shown in Table I, below, were obtained.

TABLE I
Viscosity of Uninhibited Diethylene Glycol Bis(Allyl Carbonate)

| Time (months) | Viscosity (centistokes) |
|---|---|
| 0 | 18.0 |
| 4 | 38.1 |
| 6 | 78.7 |
| 8 | 156 |
| 10 | 350 |

After ten months the composition was polymerized by adding 3.5 weight percent isopropyl peroxydicarbonate to a portion of the sample, and placing the portion of the sample between two four inch (10.2 cms) by seven inch (17.8 cms) by $\frac{1}{4}$ inch (0.7 cms) glass molds separated by a $\frac{1}{4}$ inch (0.7 cms) rubber gasket. The portion of the sample was cured according to the cure cycle shown in Table II.

TABLE II
Time-Temperature Sequence for Diisopropyl Peroxydicarbonate Cure

| Cumulative Time (hours) | Temperature, °C |
|---|---|
| 0 | 42 |
| 4 | 44 |
| 6 | 45 |
| 8 | 47 |
| 10 | 48 |
| 12 | 50 |
| 14 | 52 |
| 16 | 54.5 |
| 18 | 57 |
| 20 | 61 |
| 22 | 69 |
| 23 | 79 |
| 23.6 | 84 |
| 24 | 98 |
| 24.1 | 100 |

The resulting sheet has 3.9 percent yellowness, and a 15 second Barcol hardness of 29.

## Example II

A series of tests were conducted to determine the effect of various levels of hydroquinone monomethyl ether on a monomeric composition of diethylene glycol bis(allyl carbonate).

A composition was prepared containing 20 parts per million of hydroquinone monomethyl ether, balance PPG Industries, Inc. CR—39® diethylene glycol bis(allyl carbonate). The samples were then diluted with PPG Industries, Inc., CR—39® diethylene glycol bis(allyl carbonate) to give samples containing, respectively, 2 parts per million, 5 parts per million, and 10 parts per million of hydroquinone monomethyl ether. The four samples were then stored and tested as described in Example I, above. The results shown in Table III, below, were obtained:

TABLE III
Viscosity of Hydroquinone Monomethyl Ether Inhibited Diethylene Glycol Bis(Allyl Carbonate)

| | Amount of Hydroquinone Monomethyl Ether (parts per million) | 2 | 5 | 10 | 20 |
|---|---|---|---|---|---|
| | | Viscosity (centistokes) | | | |
| Time (months) | 0 | 17.8 | 17.7 | 17.9 | 17.6 |
| | 4 | 21.8 | 17.7 | 17.7 | 17.6 |
| | 6 | 26.3 | 18.0 | 17.4 | 17.6 |
| | 8 | 37.4 | 17.8 | 17.9 | 17.8 |
| | 10 | 54 | 17.9 | 17.9 | 17.7 |

The above viscosities are shown graphically in Figure 1, with the viscosities of the uninhibited material.

After ten months a portion of each sample was polymerized as described in Example I, above. The results obtained in Table IV, below, were obtained.

TABLE IV
Effect of Hydroquinone Monomethyl Ether on Properties of Polymerized Product

| Amount of Hydroquinone Monomethyl Ether (parts per million) | 2 | 5 | 10 | 20 |
|---|---|---|---|---|
| Yellowness (percent) | 4.1 | 3.6 | 4.2 | 4.3 |
| Barcol Hardness (15 second) | 29 | 28 | 28 | 29 |

Example III

The procedure of Example II, above, was repeated, with tertiary butyl catechol substituted for the hydroquinone monomethyl ether. The samples has the viscosities shown in Table V, below:

TABLE V
Viscosity of Tertiary-Butyl Catechol Inhibited Diethylene Glycol Bis(Allyl Carbonate)

| | Amount of Tertiary-Butyl Catechol (parts per million) | 2 | 5 | 10 | 20 |
|---|---|---|---|---|---|
| | | Viscosity (centistokes) | | | |
| Time (months) | 0 | 17.8 | 17.8 | 18.2 | 18.0 |
| | 4 | 17.8 | 17.7 | 17.7 | 17.8 |
| | 6 | 24.4 | 17.9 | 17.6 | 17.9 |
| | 8 | 37.1 | 17.7 | 17.8 | 17.9 |
| | 10 | 54.0 | 17.5 | 17.7 | 17.6 |

The data of Table V and Table I of Example I are represented in Figure 2.

The polymers cast from the samples after ten months of storage had the properties shown in Table VI below:

TABLE VI
Effect of Tertiary-Butyl Catechol on
Properties of Polymerized Product

| Amount of (parts per million) | 2 | 5 | 10 | 20 |
|---|---|---|---|---|
| Yellowness (percent) | 4.0 | 4.0 | 4.2 | 4.5 |
| Barcol Hardness (15 second) | 29 | 29 | 30 | 30 |

Example IV

The procedure of Example II, above, was repeated, with hydroquinone substituted for the hydroquinone monomethyl ether. The samples had the viscosities shown in Table VII below:

TABLE VII
Viscosity of Hydroquinone Inhibited Diethylene
Glycol Bis(Allyl Carbonate)

| | Amount of Hydroquinone (parts per million) | 2 | 5 | 10 | 20 |
|---|---|---|---|---|---|
| Time (months) | 0 | 17.7 | 17.6 | 18.0 | 17.6 |
| | 4 | 22.2 | 18.0 | 20.0 | 17.6 |
| | 6 | 26.8 | 18.2 | 17.4 | 17.4 |
| | 8 | 33.3 | 19.2 | 18.8 | 17.6 |
| | 10 | 40.9 | 20.0 | 18.0 | 17.4 |

The data of Table VII and Table I of Example I are presented in Figure 3.

The polymers cast from the samples after ten months of storage has the properties shown in Table VIII below:

TABLE VIII
Effect of Hydroquinone on Properties
of Polymerized Product

| Amount of Hydroquinone (parts per million) | 2 | 5 | 10 | 20 |
|---|---|---|---|---|
| Yellowness (percent) | 3.8 | 3.7 | 4.0 | 4.2 |
| Barcol Hardness (15 second) | 29 | 29 | 29 | 29 |

Example V

The procedure of Example II, above, was repeated with quinone substituted for the hydroquinone monomethyl ether. The samples has the viscosities shown in Table IX below:

TABLE IX
Viscosity of Quinone Inhibited Diethylene Glycol Bis(Allyl Carbonate)

| | Amount of Quinone (parts per million) | Viscosity (centistokes) 2 | 5 | 10 | 20 |
|---|---|---|---|---|---|
| Time (months) | 0 | 17.8 | 18.1 | 17.9 | 18.1 |
| | 4 | 32.4 | 27.2 | 19.9 | 17.9 |
| | 6 | 48.9 | 35.3 | 22.9 | 17.9 |
| | 8 | — | 46.4 | 27.0 | 18.1 |
| | 10 | 113.8 | 57.9 | 31.0 | 19.0 |

The data of Table IX and Table I of Example 1 are presented in Figure 4.

The polymers cast from the samples after ten months of storage had the properties shown in Table X below:

TABLE X
Effect of Quinone on Properties of Polymerized Product

| Amount of Quinone (parts per million) | 2 | 5 | 10 | 20 |
|---|---|---|---|---|
| Yellowness (percent) | 4.4 | 4.1 | 4.7 | 4.9 |
| Barcol Hardness (15 second) | 29 | 29 | 29 | 29 |

While the invention has been described with respect to specific exemplifications, embodiments, and examples, the scope is not to be limited thereby, but only by the claims appended hereto.

**Claims**

1. A composition comprising monomeric polyol(allyl carbonate) and a viscosity stabilizing amount of polymerization inhibitor chosen from the group consisting of dihydroxybenzenes, dioxo benzenes, hydroquinone monomethyl ether, hydroquinone monoethyl ether, hydroquinone benzyl ether, and mixtures thereof in the composition.

2. A composition according to claim 1 wherein the polymerization inhibitor is chosen from the group consisting of hydroquinone, mono-tertiary butyl hydroquinone, toluhydroquinone, catechol, tertiary butyl catechol, hydroquinone monomethyl ether, hydroquinone monoethyl ether, hydroquinone benzyl ether, ortho-quinone, para-quinone, toluquinone, and mixtures thereof.

3. A composition according to claim 1 or 2 wherein the composition contains from 2 to 20 parts per million of polymerization inhibitor, basis polyol(allyl carbonate).

4. A composition according to any of claims 1, 2 or 3 wherein the composition has a viscosity increase of less than ten percent at 45 degrees Centigrade measured over an interval of 10 months.

5. A method of inhibiting the thermally induced thickening of compositions containing monomeric polyol(allyl) carbonate comprising providing a viscosity stabilizing amount of a polymerization inhibitor chosen from the group consisting of dihydroxybenzenes, dioxo benzenes, hydroquinone monomethyl ether, hydroquinone monoethyl ether, hydroquinone benzyl ether, and mixtures thereof in the composition.

6. A method according to claim 5 wherein the polymerization inhibitor is chosen from the group consisting of hydroquinone, mono-tertiary butyl hydroquinone, toluhydroquinone, catechol, tertiary butyl catechol, hydroquinone monomethyl ether, hydroquinone monoethyl ether, hydroquinone benzyl ether, orthoquinone, para-quinone, toluquinone, and mixtures thereof.

7. A method according to claim 5 or 6 wherein the composition contains from 2 to 20 parts per million

of polymerization inhibitor, basis polyol(allyl carbonate).

8. A method according to any of claims 5 to 7 wherein the composition has a viscosity increase of less than ten percent at 45 degrees Centigrade measured over an interval of 10 months.

9. A method of polymerizing a composition of polyol(allyl carbonate) monomer comprising providing a viscosity stabilizing amount of a polymerization inhibitor chosen from the group consisting of dihydroxybenzenes, dioxo benzenes, hydroquinone monomethyl ether, hydroquinone monoethyl ether, hydroquinone benzyl ether, and mixtures thereof in the composition whereby to inhibit the thermally induced thickening thereof, and thereafter, without removing the polymerization inhibitor, adding a free radical polymerization initiator to the composition and heating the composition whereby to polymerize the composition.

10. A method according to claim 9 wherein the polymerization inhibitor is chosen from the group consisting of hydroquinone, mono-tertiary butyl hydroquinone, toluhydroquinone, catechol, tertiary butyl catechol, hydroquinone monomethyl ether, hydroquinone monoethyl ether, hydroquinone benzyl ether, orthoquinone, para-quinone, toluquinone, and mixtures thereof.

11. A method according to claim 9 or 10 wherein the composition contains from 2 to 20 parts per million of polymerization inhibitor, basis polyol(allyl carbonate).

12. A method according to any of claims 9 to 11 wherein, prior to the addition of the free radical polymerization initiator, the composition of polyol(allyl carbonate) monomer has a viscosity increase of less than ten percent at 45 degrees Centigrade measured over an interval of 10 months.

**Patentansprüche**

1. Zusammensetzung enthaltend ein monomeres Polyol-(allylcarbonat) und eine die Viskosität stabilisierende Menge eines Polymerisationshemmers aus der Gruppe Dihydroxybenzole, Dioxobenzole, Hydrochinonmonomethylether, Hydrochinonmonoethylether, Hydrochinonbenzylether und Mischungen derselben.

2. Zusammensetzung nach Anspruch 1, dadurch gekennzeichnet, daß der Polymerisationshemmer aus der Gruppe ist, aus Hydrochinon, Mono-tertiär-butylhydrochinon, Toluhydrochinon, Brenzkatechin, Tertiär-butylbrenzkatechin, Hydrochinonmonomethylether, Hydrochinonmonoethylether, Hydrochinonbenzylether, o-Chinon, p-Chinon, Toluchinon und Mischungen derselben.

3. Zusammensetzung nach Anspruch 1 oder 2, dadurch gekennzeichnet, daß sie 2 bis 20 Teile pro Million bezogen auf Polyol-(allylcarbonat) Polymerisationshemmer enthält.

4. Zusammensetzung nach jedem der Ansprüche 1, 2 oder 3, dadurch gekennzeichnet, daß die Zusammensetzung während einer Zeit von 10 Monaten einen Viskositätsanstieg von weniger als 10% bei 45°C aufweist.

5. Verfahren zum Hemmen der durch Wärme ausgelösten Verdickung einer Zusammensetzung eines monomeren Polyol-(allylcarbonat) mittels einer die Viskosit t stabilisierenden Menge eines Polymerisationshemmers aus der Gruppe Dihydroxybenzole, Dioxobenzole, Hydrochinonmonomethylether, Hydrochinonmonoethylether, Hydrochinonbenzylether und Mischungen derselben.

6. Verfahren nach Anspruch 5, dadurch gekennzeichnet, daß der Polymerisationshemmer ausgewählt ist aus Hydrochinon, Mono-tertiär-butylhydrochinon, Toluhydrochinon, Brenzkatechin, Tertiär-butylbrenzkatechin, Hydrochinonmonomethylether, Hydrochinonmonoethylether, Hydrochinonbenzylether, o-Chinon, p-Chinon, Toluchinon und Mischungen derselben.

7. Verfahren nach Anspruch 5 oder 6, dadurch gekennzeichnet, daß die Zusammensetzung 2 bis 20 Teile pro Million bezogen auf Polyol-(allylcarbonat) Polymerisationshemmer enthält.

8. Verfahren nach jedem der Ansprüche 5 bis 7, dadurch gekennzeichnet, daß die Zusammensetzung während einer Zeit von 10 Monaten einen Viskositätsanstieg von weniger als 10% bei 45°C aufweist.

9. Verfahren zum Polymerisieren einer Zusammensetzung aus monomeren Polyol-(allylcarbonat), gekennzeichnet durch Zugeben einer die Viskosität stabilisierenden Menge eines Polymerisationshemmers aus der Gruppe Dihydroxybenzole, Dioxybenzole, Hydrochinonmonomethylether, Hydrochinonmonoethylether, Hydrochinonbenzylether und Mischungen derselben, so daß das durch Wärme ausgelöste Verdicken gehemmt ist und anschließendes Zugeben einer die freie radikalische Polymerisation auslösenden Verbindung, ohne Entfernung des Polymerisationshemmers und Erwärmen der Zusammensetzung, so daß diese polymerisiert.

10. Verfahren nach Anspruch 9, dadurch gekennzeichnet, daß der Polymerisationshemmer ausgewählt ist aus Hydrochinon, Mono-tertiär-butylhydrochinon, Toluhydrochinon, Brenzkatechin, Tertiär-butylbrenzkatechin, Hydrochinonmonomethylether, Hydrochinonmonoethylether, Hydrochinonbenzylether, o-Chinon, p-Chinon, Toluchinon und Mischungen derselben.

11. Verfahren nach Anspruch 9 oder 10, dadurch gekennzeichnet, daß die Zusammensetzung 2 bis 20 Teile pro Million bezogen auf Polyol-(allylcarbonat) Polymerisationshemmer enthält.

12. Verfahren nach jedem der Ansprüche 9 bis 11, dadurch gekennzeichnet, daß die Zusammensetzung des Polyol-(allylcarbonat) Monomeren vor der Zugabe des die freie radikalische Polymerisation auslösenden Stoffes während einer Zeit von 10 Monaten einen Viskositätsanstieg von weniger als 10% bei 45°C aufweist.

**Revendications**

1. Composition comprenant un monomère de type (allylcarbonate) de polyol et une quantité, capable de stabiliser la viscosité, d'un inhibiteur de polymérisation choisi dans l'ensemble constitué par les dihydroxybenzènes, les dioxo benzènes, l'éther monométhylique de l'hydroquinone, l'éther monoéthylique de l'hydroquinone, l'éther benzylique de l'hydroquinone, et leurs mélanges, introduit dans la composition.

2. Composition selon la revendication 1, dans laquelle l'inhibiteur de polymérisation est choisi dans l'ensemble constitué par l'hydroquinone, la monotertiobutyl hydroquinone, la tolyhydroquinone, le catéchol, le tertiobutyl catéchol, l'éther monométhylique de l'hydroquinone, l'éther monoéthylique de l'hydroquinone, l'éther benzylique de l'hydroquinone, l'ortho-quinone, la para-quinone, la toluquinone, et leurs mélanges.

3. Composition selon la revendication 1 ou 2, dans laquelle la composition contient de 2 à 20 parties par million de l'inhibiteur de polymérisation, sur la base de l'(allylcarbonate) de polyol.

4. Composition selon l'une quelconque des revendications 1, 2 ou 3, dans laquelle la composition présente une augmentation de sa viscosité inférieure à 10% à 45 degrés centigrades, quand on effectue la mesure pour un intervalle de dix mois.

5. Procédé pour inhiber l'épaississement, par la chaleur de compositions contenant de l'(allylcarbonate) de polyol monomère, consistant à introduire dans la composition une quantité stabilisant la viscosité, d'un inhibiteur de polymérisation choisi dans l'ensemble constitué par les dihydroxybenzènes, les dioxo benzènes, l'éther monométhylique de l'hydroquinone, l'éther monoéthylique de l'hydroquinone, l'éther benzylique de l'hydroquinone, et leurs mélanges.

6. Procédé selon la revendication 5, dans lequel l'inhibiteur de polymérisation est choisi parmi l'ensemble constitué par l'hydroquinone, la mono-tertiobutyl hydroquinone, la toluhydroquinone, le catéchol, le tertiobutyl catéchol, l'éther monométhylique de l'hydroquinone, l'éther monoéthylique de l'hydroquinone, l'éther benzylique de l'hydroquinone, l'ortho-quinone, la para-quinone, la toluquinone et leurs mélanges.

7. Procédé selon la revendication 5 ou 6, dans lequel la composition contient de 2 à 20 parties par million de l'inhibiteur de polymérisation, sur la base de l'(allylcarbonate) de polyol.

8. Procédé selon l'une quelconque des revendications 5 à 7, dans lequel la composition présente une augmentation de sa viscosité inférieure à 10% à 45 degrés centigrades, quand la mesure est effectuée pour un intervalle de dix mois.

9. Procédé pour polymériser une composition de (allyl carbonate) de polyol monomère, consistant à introduire dans la composition une quantité, capable de stabiliser la viscosité, d'un inhibiteur de polymérisation choisi dans l'ensemble constitué par les dihydroxybenzènes, les dioxobenzènes, l'éther monométhylique de l'hydroquinone, l'éther monoéthylique de l'hydroquinone, l'éther benzylique de l'hydroquinone, et leurs mélanges, de façon à inhiber l'épaississement par la chaleur de la composition, puis, sans enlever l'inhibiteur de polymérisation, à ajouter à la composition un amorceur de polymérisation par radicaux libres et à chauffer la composition de façon à polymériser celle-ci.

10. Procédé selon la revendication 9, dans lequel l'inhibiteur de polymérisation est choisi dans l'ensemble constitué par l'hydroquinone, la mono-tertiobutyl hydroquinone, la toluhydroquinone, le catéchol, le tertiobutyl catéchol, l'éther monométhylique de l'hydroquinone, l'éther monoéthylique de l'hydroquinone, l'éther benzylique de l'hydroquinone, l'ortho-quinone, la para-quinone, la toluquinone et leurs mélanges.

11. Procédé selon la revendication 9 ou la 10, dans lequel la composition contient de 2 à 20 parties par million de l'inhibiteur de polymérisation, sur la base de l'(allylcarbonate) de polyol.

12. Procédé selon l'une quelconque des revendications 9 à 11, dans lequel, avant l'addition de l'amorceur de polymérisation par radicaux libres, la composition de (allylcarbonate) de polyol monomère présente une augmentation de viscosité inférieure à 10% à 45 degrés centigrades, quand on effectue la mesure pour un intervalle de dix mois.

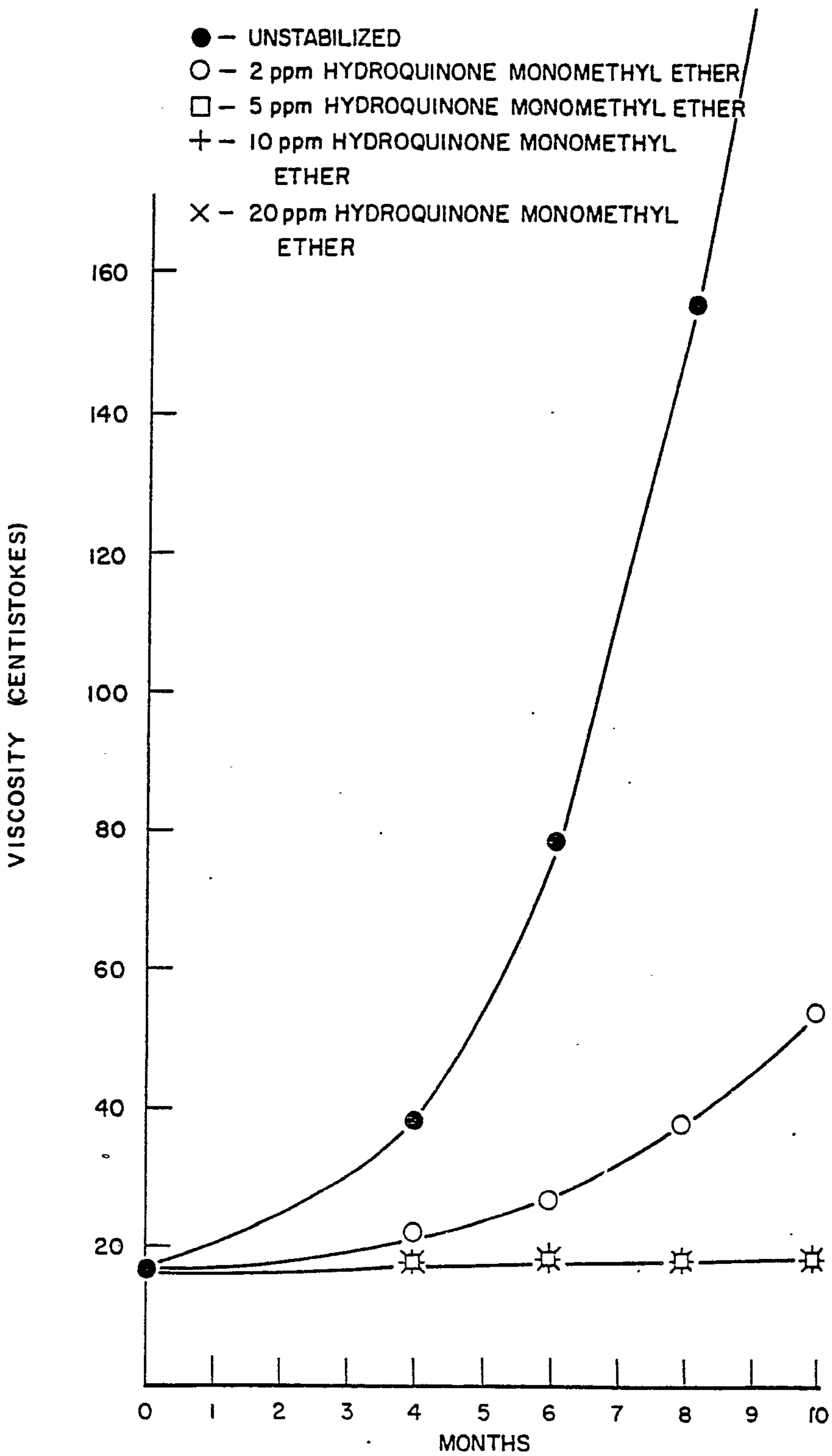
● – UNSTABILIZED
○ – 2 ppm HYDROQUINONE MONOMETHYL ETHER
□ – 5 ppm HYDROQUINONE MONOMETHYL ETHER
+ – 10 ppm HYDROQUINONE MONOMETHYL ETHER
× – 20 ppm HYDROQUINONE MONOMETHYL ETHER
VISCOSITY (CENTISTOKES)
160
140
120
100
80
60
40
20
0
1
2
3
4
5
6
7
8
9
10
MONTHS

FIG. 1

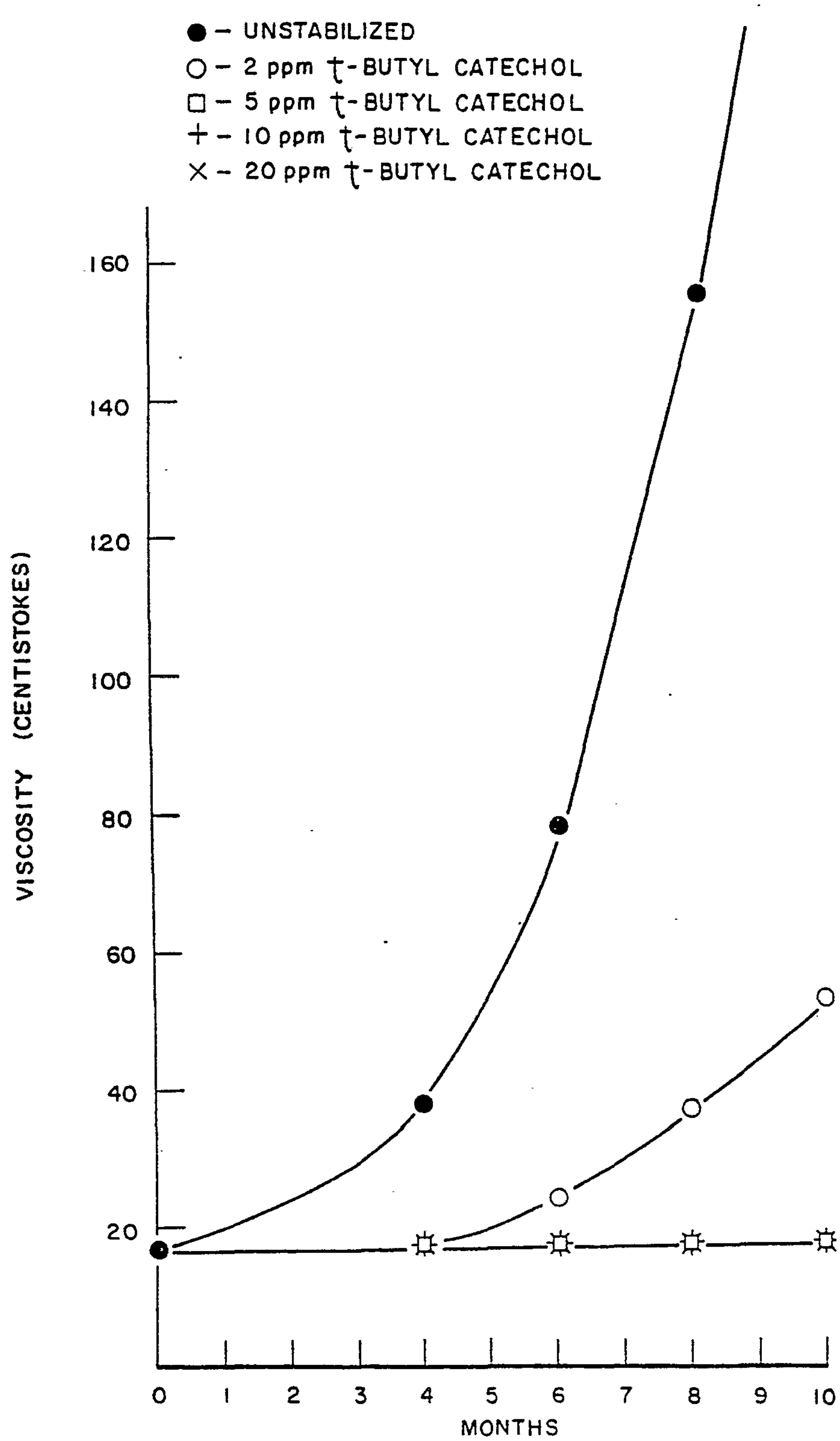
● – UNSTABILIZED
○ – 2 ppm t-BUTYL CATECHOL
□ – 5 ppm t-BUTYL CATECHOL
+ – 10 ppm t-BUTYL CATECHOL
× – 20 ppm t-BUTYL CATECHOL
VISCOSITY (CENTISTOKES)
160
140
120
100
80
60
40
20
0
1
2
3
4
5
6
7
8
9
10
MONTHS

FIG. 2

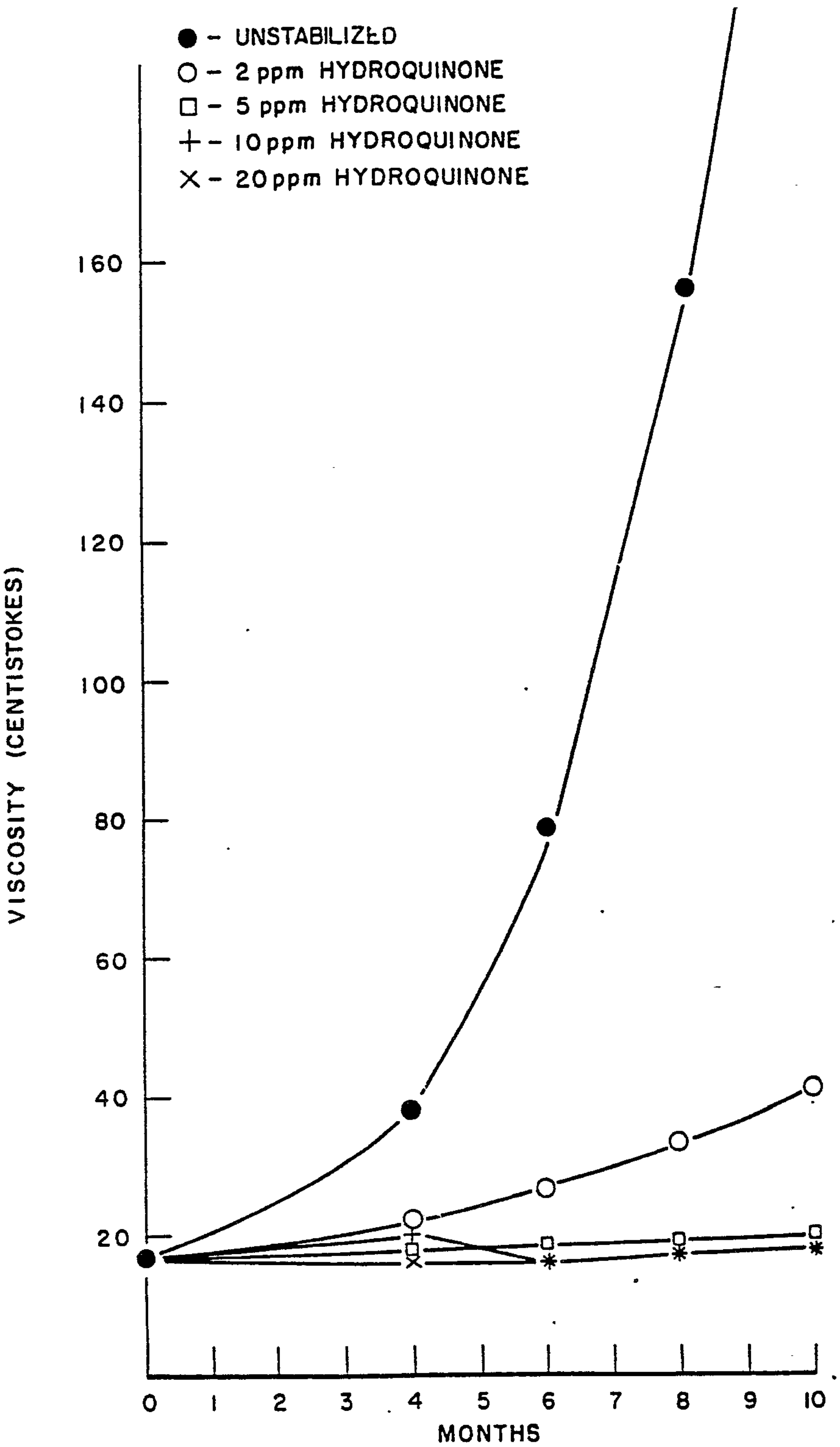
● - UNSTABILIZED
○ - 2 ppm HYDROQUINONE
□ - 5 ppm HYDROQUINONE
+ - 10 ppm HYDROQUINONE
× - 20 ppm HYDROQUINONE
VISCOSITY (CENTISTOKES)
160
140
120
100
80
60
40
20
0
1
2
3
4
5
6
7
8
9
10
MONTHS

FIG. 3

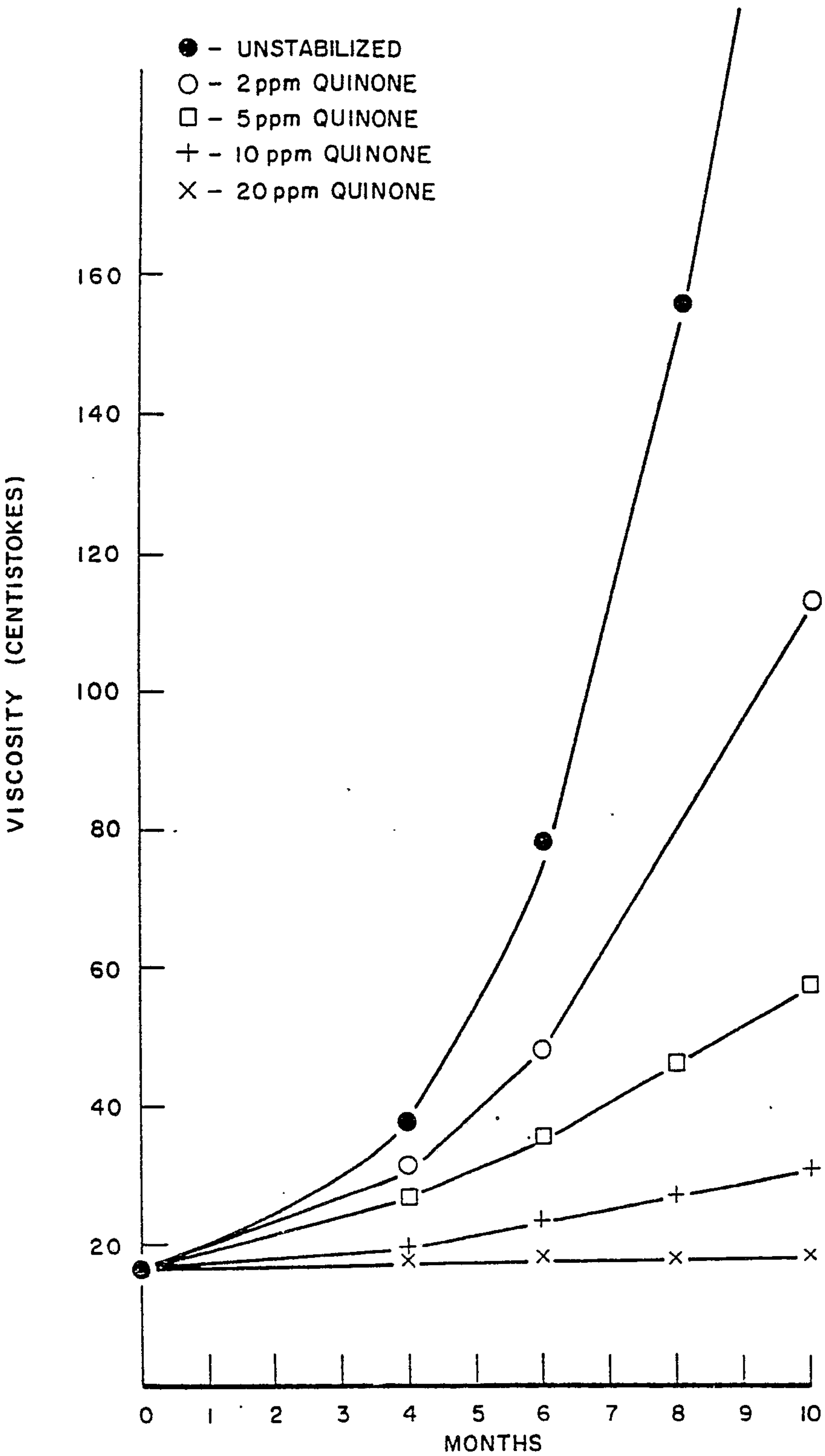
● - UNSTABILIZED
○ - 2 ppm QUINONE
□ - 5 ppm QUINONE
+ - 10 ppm QUINONE
× - 20 ppm QUINONE
VISCOSITY (CENTISTOKES)
160
140
120
100
80
60
40
20
0
1
2
3
4
5
6
7
8
9
10
MONTHS

FIG. 4